# FASCICULE DE BREVET EUROPEEN

(11) **EP 0 335 767 B1**
(45) Date de publication et mention de la délivrance du brevet: **02.08.1995**
(21) Numéro de dépôt: 89400753.3
(22) Date de dépôt: 17.03.1989
(51) Int. Cl.: A61B 5/05

(54) **Dispositif pour réaliser une mesure de la teneur en eau d'un substrat, notamment de la peau**
Vorrichtung zur Messung des Wassergehaltes der Haut
Device for measuring the water content of skin

(30) Priorité: 25.03.1988 FR 8803937
(43) Date de publication de la demande: 04.10.1989
(73) Titulaire: L'OREAL, 75008 Paris (FR)
(72) Inventeur: Scot, Jean, F-75018 Paris (FR); Bazin, Roland, F-94400 Vitry S/Seine (FR); Leveque, Jean-Luc, F-93340 Le Raincy (FR)
(74) Mandataire: Peuscet, Jacques

(56) Documents cités:
- DE-A- 1 598 910
- FR-A- 1 265 357
- FR-A- 2 266 882
- MEDICAL & BIOLOGICAL ENGINEERING & COMPUTING, vol. 24, no. 1, janvier 1986, pages 71-77, IFMBE, Stevenage, Herts, GB; Y. YAMAMOTO et al.: "Characte-ristics of skin admittance for dry electrodes and the measurement of skin mois- turisation"

## Description

L'invention est relative à un dispositif pour réaliser une mesure de la teneur en eau d'un substrat, du genre de ceux qui comprennent un générateur de signaux alternatifs du type haute fréquence dont la sortie est reliée à un embout de sonde destiné à être appliqué contre le substrat, un amplificateur dont l'entrée est reliée à l'embout de sonde, et des moyens de détection propres à établir un signal de tension continue, à partir du signal de sortie de l'amplificateur, le niveau de ce signal de tension continue dépendant de la teneur en eau du substrat.

L'invention concerne plus particulièrement, parce que c'est dans ce cas que son application semble devoir présenter le plus d'intérêt, mais non exclusivement, un tel dispositif pour réaliser une mesure de la teneur en eau de la peau humaine.

Un dispositif de ce genre est connu d'après l'article intitulé "Characteristics of skin admittance for dry electrodes and the measurement of skin moisturisation" de Y. YAMAMOTO et al. paru dans MEDICAL & BIOLOGICAL ENGINEERING & COMPUTING, vol. 24, n° 1, janvier 1986, pages 71-77 IFMBE, Stevenage, Herts, GB. Dans ce dispositif de l'art antérieur, l'oscillateur du générateur de signaux, constituant un boîtier fixe, est relié à une électrode d'un embout de sonde par un câble coaxial.

La présence d'un tel câble coaxial, nécessaire pour conduire les signaux haute fréquence engendre un certain nombre d'inconvénients. La manipulation d'une électrode reliée par un câble coaxial, relativement rigide, à un boîtier fixe n'est pas d'une grande souplesse. L'utilisation d'un tel câble coaxial pour la transmission d'un signal nécessite l'adaptation de la ligne par une résistance qui dissipe de l'énergie avant que le signal n'arrive à l'élément de détection, ce qui réduit la sensibilité de l'appareil.

En outre, une électrode avec son câble coaxial fait partie de la capacité du montage de mesure dont les valeurs sont données pour une fréquence de l'oscillateur, par exemple 100 KHz. La longueur du câble coaxial est donc imposée. Si l'on souhaite modifier la longueur du câble coaxial, par exemple pour une application différente nécessitant d'éloigner davantage l'électrode du boîtier fixe, l'appareil devra être adapté et réétalonné. Si l'on souhaite modifier la fréquence de l'oscillateur, l'adaptation du système doit être modifiée en conséquence, avec des répercussions possibles au niveau de la longueur du câble coaxial.

L'invention a pour but, notamment, de fournir un dispositif dans lequel il n'est pas nécessaire d'utiliser des câbles coaxiaux pour les liaisons électriques.

Un autre but de l'invention est de réduire sensiblement les écarts de mesure entre deux appareils pour un même substrat. Autrement dit, on souhaite obtenir une bonne correspondance entre la teneur en eau du substrat et le résultat de la mesure donné par le dispositif.

Un autre but de l'invention est de fournir un dispositif qui permette d'effectuer d'une manière simple la mesure, avec une sonde facile à mettre en oeuvre, et qui prenne en compte la teneur en eau suivant une profondeur non négligeable du substrat.

Selon l'invention, un dispositif pour réaliser une mesure de la teneur en eau d'un substrat, notamment de la peau, du genre défini précédemment, est caractérisé par le fait que l'ensemble du générateur de tension, de l'amplificateur et des moyens de détection est logé à l'intérieur d'une enveloppe métallique et qu'un écran au rayonnement électromagnétique est disposé dans l'enveloppe pour séparer et protéger l'amplificateur et les moyens de détection du générateur, l'embout de sonde étant prévu à une extrémité de l'enveloppe.

La fréquence du générateur est supérieure à 100 KHz, et est de préférence de l'ordre de 10,7 MHz.

Avantageusement, le générateur de signaux est un générateur de tension de sorte que son signal de sortie, notamment en ce qui concerne l'amplitude et la fréquence, ne soit pas perturbé par une variation de la charge sur laquelle débite le générateur.

De préférence, le générateur de tension est relié, à sa sortie, par une résistance, à l'embout de la sonde : la susdite résistance est choisie suffisamment grande pour isoler correctement le générateur de tension, et suffisamment faible pour permettre d'obtenir un signal suffisant à l'entrée de l'amplificateur.

Cette résistance est avantageusement de l'ordre de 1 KΩ.

L'amplificateur est choisi avec une forte impédance d'entrée pour ne pas perturber la mesure. Cette impédance d'entrée est avantageusement de l'ordre de 100 KΩ (100 kiloohms).

L'enveloppe métallique dans laquelle sont logés le générateur de tension, l'amplificateur et les moyens de détection, a, de prérence une forme cylindrique de révolution ; l'écran au rayonnement électromagnétique, disposé dans cette enveloppe, est constitué par une plaque métallique.

Avantageusement, cette plaque est située dans un plan diamétral du cylindre, et divise le volume intérieur de l'enveloppe en deux demicylindres, la générateur haute fréquence étant placé dans un de ces demi-cylindres, tandis que l'amplificateur et les moyens de détection sont situés dans l'autre demi-cylindre.

L'invention consiste, mises à part les dispositions exposées ci-dessus, en un certain nombre d'autres dispositions dont il sera plus explicitement question ci-après à propos d'un mode de réalisation particulier décrit avec référence au dessin ci-annexé, mais qui n'est nullement limitatif.

La figure 1, de ce. dessin, est une coupe longitudinale schématique d'un dispositif conforme à l'invention.

La figure 2, enfin, est une coupe suivant la ligne II-II figure 1.

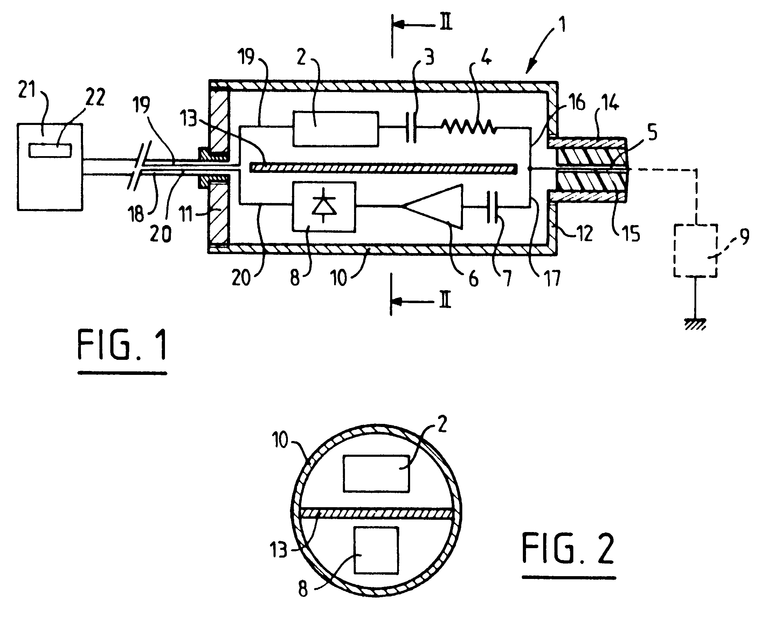

En se reportant à la figure 1 des dessins, on peut voir un dispositif 1 pour réaliser une mesure de la teneur en eau d'un substrat, notamment de la peau humaine.

Ce dispositif comprend un générateur 2 de signaux alternatifs à haute fréquence, notamment supérieure à 100 KHz,et de préférence égale à 10,7 MHz. La sortie de ce générateur 2 est reliée, par l'intermédiaire d'un condensateur 3 et d'une résistance 4, à un embout de sonde 5, constitué par une tige ou pointe métallique, destiné à être appliqué contre le substrat.

Le générateur 2 est un générateur de tension de sorte que le signal de sortie, fourni par ce générateur, notamment en ce qui concerne l'amplitude et la fréquence, ne soit pas perturbé par une variation de la charge sur laquelle le générateur 2 débite.

Le dispositif 1 comprend également un amplificateur 6 dont l'entrée est reliée, par l'intermédiaire d'un condensateur 7, à l'embout de sonde 5. L'amplificateur 6 a une forte impédance d'entrée, notamment de l'ordre de 100 KΩ, pour ne pas perturber la mesure.

Des moyens de détection 8, dont l'entrée est reliée à la sortie de l'amplificateur 6, sont prévus pour établir un signal de tension continue, sur leur sortie, à partir des signaux provenant de l'amplificateur.

Lorsque l'embout de sonde 5 est appliqué contre le substrat, en particulier la peau, du point de vue électrique, l'extrémité de la pointe 5 se trouve reliée à la masse par l'intermédiaire d'une impédance 9 dont la valeur dépend de la teneur en eau du substrat.

Le niveau du signal de tension continue, obtenu à la sortie des moyens de détection 8, dépend de la valeur de l'impédance 9 et donc de la teneur en eau du substrat.

L'ensemble du générateur de tension 2, de l'amplificateur 6 et des moyens de détection 8 est logé à l'intérieur d'une enveloppe métallique 10, de forme cylindrique de révolution, fermée à ses deux extrémités longitudinales par une paroi transversale 11, 12 également métallique.

Un écran 13 au rayonnement électromagnétique est disposé dans l'enveloppe 10 pour séparer et protéger l'amplificateur 6 et les moyens de détection 8, vis-à-vis du générateur haute fréquence 2. Cet écran 13 est constitué par une plaque métallique, disposée et fixée suivant un plan diamétral de l'enveloppe métallique 10, laquelle se trouve ainsi divisée en deux demi-cylindres. Le générateur 2 est situé dans un de ces demi-cylindres, tandis que l'amplificateur 6 et les moyens de détection 8 sont situés dans l'autre demi-cylindre.

L'enveloppe 10 et l'écran 13 peuvent être réalisés en un alliage léger, notamment à base d'aluminium.

La tige formant l'embout de sonde 5 est prévue à une extrémité de l'enveloppe 10 qu'elle prolonge suivant son axe, orthogonalement à la paroi 12. Cette paroi est munie d'un prolongement cylindrique 14 faisant saillie vers l'extérieur, entourant la tige 5. Un manchon isolant 15 en matière plastique, notamment en polytétrafluoroéthylène, est prévu pour entourer la tige 5 à l'intérieur du prolongement 14. L'extrémité intérieure de la tige 5 est reliée, d'un côté de l'écran 13 à la résistance 4 et, de l'autre côté dudit écran 13, à une borne du condensateur 7, par un conducteur électrique respectivement 16, 17 de longueur réduite. Il n'est pas nécessaire d'utiliser, pour ces liaisons électriques, de câbles coaxiaux, en raison de la configuration particulière avec écran 13 séparateur et de la longueur réduite des conducteurs.

La résistance 4 est choisie de manière que l'on obtienne la meilleure réponse à l'entrée de l'amplificateur 6, cette résistance 4 étant suffisamment grande pour isoler de manière satisfaisante le générateur 2, et suffisamment faible pour permettre d'obtenir un signal utile suffisant à l'entrée de l'amplificateur 6. Cette résistance 4 est avantageusement de l'ordre de 1 KΩ (1 kiloohm) .

La paroi transversale 11, opposée à celle équipée de la tige 5, est traversée dans sa région centrale par un câble 18 comprenant un conducteur 19 pour assurer l'alimentation en énergie, en particulier du générateur 2, et un conducteur 20 pour transmettre le signal de sortie fourni par les moyens de détection 8. Ce câble 18 est relié à un ensemble 21 comprenant une source d'énergie pour alimenter l'ensemble des circuits contenus dans l'enveloppe 1 et des moyens d'affichage 22, de préférence sous forme digitale, du niveau du signal obtenu à la sortie des moyens de détection 8.

La valeur numérique apparaissant sur ces moyens d'affichage constitue le résultat de la mesure proprement dite.

Des moyens (non représentés) sont prévus pour permettre d'appliquer l'embout 5 selon une pression constante contre le substrat, avec déclenchement automatique de la mesure lorsque la pression d'application souhaitée est obtenue. Ces moyens peuvent comprendre une jauge de contrainte placée sur le prolongement 14, ou un montage coulissant du prolongement 14 et de l'embout 5 dans l'enveloppe 10, avec interposition de moyens élastiques dont la compression détermine la force d'application souhaitée.

On peut prévoir dans l'enveloppe 10, en vue d'un interfacage avec un ordinateur, un circuit oscillateur piloté par l'amplitude du signal à la sortie des moyens de détection 8, la sortie de ce circuit oscillateur étant reliée par un câble à l'ordinateur. La période du signal fourni par le circuit oscillateur constitue l'élément de mesure pris en compte par l'ordinateur. L'intervalle de temps entre deux fronts de montée du signal fourni par le circuit oscillateur constitue une image de la tension à la sortie des moyens de détection 8.

Ceci étant, le fonctionnement du dispositif est le suivant.

L'appareil ayant été mis en état de fonctionnement, l'ensemble 21 assure l'alimentation en énergie des circuits de l'enveloppe 10.

L'utilisateur saisit alors l'enveloppe 10 d'une main et applique l'embout 5 contre la peau sur laquelle la mesure doit être effectuée, suivant une direction sensiblement orthogonale à la surface de cette peau. L'utilisateur applique sur l'enveloppe 10 une force suffisante pour obtenir la pression d'application souhaitée de l'embout 5 sur la peau.

La lecture de la mesure est réalisée sur les moyens d'affichage 22 de l'ensemble 21.

Des essais effectués avec un générateur de fréquence variable, en trempant l'embout de sonde 5 dans une saumure, ont montré que la fréquence de 10,7 MHz donne une dynamique de mesure importante. Cette fréquence élevée permet d'effectuer une mesure en profondeur de la teneur en eau du substrat.

La séparation physique métallique entre le générateur 2 d'une part et l'amplificateur 6 et les moyens de détection 8 d'autre part, permet de réaliser un isolement radio-électrique entre ces composants et évite toute perturbation de l'amplificateur et des moyens de détection par un rayonnement du générateur 2.

En jouant sur les réglages de gain du générateur 2 et de l'amplificateur 6, on peut avoir une même courbe de réponse pour différents appareils.

Des mesures réalisées avec des appareils différents sur des saumures ont permis d'obtenir des résultats très voisins, à un pour cent près environ.

La forte impédance d'entrée de l'amplificateur 6 permet d'éviter des perturbations sur le générateur 2.

L'embout de sonde 5 qui fait intervenir une simple tige ou pointe est d'un encombrement réduit et d'une utilisation particulièrement simple.

La disposition des circuits à l'intérieur de l'enveloppe 10, avec séparation par l'écran 13, permet de s'affranchir de l'utilisation d'un câble coaxial avec blindage pour relier l'embout de sonde 5 au générateur 2 et à l'amplificateur 6.

Suivant une variante de la présente invention, les dispositifs d'alimentation 21 et d'affichage 22 peuvent être contenus dans la tête de mesure ce qui permet d'obtenir un système portable.

## Revendications

1. Dispositif pour réaliser une mesure de la teneur en eau d'un substrat, notamment de la peau, comprenant un générateur (2) de signaux alternatifs du type haute fréquence dont la sortie est reliée à un embout de sonde (5) destiné à être appliqué contre le substrat, un amplificateur (6) dont l'entrée est reliée à l'embout de sonde, et des moyens de détection (8) propres à établir un signal de tension continue à partir du signal de sortie de l'amplificateur, le niveau du signal de tension continue dépendant de la teneur en eau du substrat, caractérisé par le fait que l'ensemble du générateur (2), de l'amplificateur (6) et des moyens de détection (8) est logé à l'intérieur d'une enveloppe métallique (10) et qu'un écran (13) au rayonnement électromagnétique est disposé dans l'enveloppe (10) pour séparer et protéger l'amplificateur (6) et les moyens de détection (8) du générateur (2), l'embout de sonde (5) étant prévu à une extrémité de l'enveloppe (10).

2. Dispositif selon la revendication 1, caractérisé par le fait que la fréquence du générateur est supérieure à 100 KHZ.

3. Dispositif selon la revendication 1 ou 2, caractérisé par le fait que la fréquence du générateur est de l'ordre de 10,7 MHz.

4. Dispositif selon l'une des revendications précédentes, caractérisé par le fait que le générateur (2) est un générateur de tension de sorte que le signal de sortie de ce générateur ne soit pas perturbé, en particulier en ce qui concerne l'amplitude et la fréquence, par une variation de la charge sur laquelle ce générateur débite.

5. Dispositif selon la revendication 4, caractérisé par le fait que le générateur de tension (2) est associé, sur sa sortie, à une résistance (4) choisie de telle manière que l'on obtienne la meilleure réponse à l'entrée de l'amplificateur (6), cette résistance (4) étant suffisamment grande pour isoler de manière satisfaisante le générateur de tension (2) et suffisamment faible pour permettre d'obtenir un signal utile suffisant à l'entrée de l'amplificateur (6).

6. Dispositif selon la revendication 5, caractérisé par le fait que la résistance (4) reliée à la sortie du générateur a une valeur de l'ordre de 1 KΩ (1 kiloohm).

7. Dispositif selon l'une des revendications précédentes, caractérisé par le fait que l'amplificateur (6) a une forte impédance d'entrée, notamment de l'ordre de 100 KΩ (100 kiloohms).

8. Dispositif selon l'une des revendications précédentes, caractérisé par le fait que l'enveloppe métallique (10) a une forme cylindrique et que l'écran (13) au rayonnement électromagnétique, disposé dans cette enveloppe, est constitué par une plaque métallique.

9. Dispositif selon la revendication 8, caractérisé par le fait que la plaque métallique (13) est située dans un plan diamétral du cylindre et divise le volume intérieur de l'enveloppe (10) en deux demicylindres, le générateur (2) étant placé dans un de ces demi-cylindres, tandis que l'amplificateur (6) et les moyens de détection (8) sont situés dans l'autre demicylindre.

## Claims

1. Device for taking a measurement of the water content of a substrate, in particular of the skin, comprising a high frequency-type AC signal generator (2) the output of which is connected to a probe end-piece (5) intended to be applied against the substrate, an amplifier (6) the input of which is connected to the probe end-piece, and detection means (8) capable of establishing a DC voltage signal from the output signal of the amplifier, the level of the DC voltage signal depending on the water content of the substrate, characterized in that the assembly comprising the generator (2), the amplifier (6) and the detection means (8) is housed inside a metallic casing (10); and in that an electromagnetic-radiation screen (13) is arranged in the casing (10) in order to separate and protect the amplifier (6) and the detection means (8) from the generator (2), the probe end-piece (5) being provided at one end of the casing (10).

2. Device according to Claim 1, characterized in that the frequency of the generator is greater than 100 kHz.

3. Device according to Claim 1 or 2, characterized in that the frequency of the generator is of the order of 10.7 MHz.

4. Device according to one of the preceding claims, characterized in that the generator (2) is a voltage generator, so that the output signal of this generator is not disturbed, in particular as regards amplitude and frequency, by a variation in the load to which this generator delivers.

5. Device according to Claim 4, characterized in that the voltage generator (2) is associated, on its output, with a resistor (4) chosen so that the best input response of the amplifier (6) is obtained, this resistor (4) having a resistance which is sufficiently high to isolate the voltage generator (2) satisfactorily and sufficiently low to make it possible to obtain a sufficient useful signal at the input of the amplifier (6).

6. Device according to Claim 5, characterized in that the resistor (4) connected to the output of the generator has a resistance of the order of 1 kΩ (1 kilohm).

7. Device according to one of the preceding claims, characterized in that the amplifier (6) has a high input impedance, in particular of the order of 100 kΩ (100 kilohms).

8. Device according to one of the preceding claims, characterized in that the metallic casing (10) has a cylindrical shape; and in that the electromagnetic-radiation screen (13) arranged in this casing consists of a metallic plate.

9. Device according to Claim 8, characterized in that the metallic plate (13) is located in a diametral plane of the cylinder and divides the internal volume of the casing (10) into two hemicylinders, the generator (2) being placed in one of these hemicylinders whereas the amplifier (6) and the detection means (8) are located in the other hemicylinder.

## Patentansprüche

1. Vorrichtung zum Messen des Wassergehaltes eines Substrates, insbesondere der Haut, umfassend einen Generator (2) für hochfrequente Wechselsignale, dessen Ausgang mit einem, zum Ansetzen an das Substrat bestimmten Meßkopf (5) verbunden ist, einen Verstärker (6), dessen Eingang mit dem Meßkopf verbunden ist, und Detektionsmittel (8), die aus dem Ausgangssignal des Verstärkers ein Gleichspannunssignal erzeugen können, wobei die Höhe des Gleichspannungssignals von dem Wassergehalt des Substrates abhängt, dadurch gekennzeichnet, daß sich die Anordnung von Generator (2), Verstärker (6) und Detektionsmitteln (8) im Inneren eines Metallgehäuses (10) befindet, und daß eine Abschirmung (13) für elektromagnetische Strahlung in dem Gehäuse (10) angeordnet ist, um den Verstärker (6) und die Detektionsmittel (8) vom Generator (2) zu trennen und zu schützen, wobei der Meßkopf (5) an einem Ende des Gehäuses (10) vorgesehen ist.

2. Vorrichtung gemäß Anspruch 1, dadurch gekennzeichnet, daß die Generatorfrequenz höher als 100 KHz ist.

3. Vorrichtung gemäß Anspruch 1 oder 2, dadurch gekennzeichnet, daß die Generatorfrequenz in der Größenordnung von 10,7 MHz liegt.

4. Vorrichtung gemäß einem der vorhergehenden Ansprüche, dadurch gekennzeichnet, daß der Generator (2) ein solcher Spannungsgenerator ist, daß das Ausgangssignal des Generators nicht durch eine Änderung der Last gestört wird, die dieser Generator liefert, insbesondere nicht in Bezug auf die Amplitude und die Frequenz.

5. Vorrichtung gemäß Anspruch 4, dadurch gekennzeichnet, daß der Spannungsgenerator (2) an seinem Ausgang mit einem Widerstand (4) verbunden ist, der so gewählt ist, daß eine bessere Antwort am Eingang des Verstärkers (6) erhältlich ist, wobei der Widerstand (4) groß genug für eine ausreichende Isolierung des Spannungsgenerators (2) und klein genug für den Erhalt eines ausreichenden Nutzsignals am Eingang des Verstärkers (6) ist.

6. Vorrichtung gemäß Anspruch 5, dadurch gekennzeichnet, daß der mit dem Generatorausgang verbundene Widerstand (4) einen Wert in der Größenordnung von 1 KΩ (1 Kiloohm) besitzt.

7. Vorrichtung gemäß einem der vorhergehenden Ansprüche, dadurch gekennzeichnet, daß der Verstärker (6) eine hohe Eingangsimpedanz, insbesondere in der Größenordnung von 100 KΩ (100 Kiloohm) besitzt.

8. Vorrichtung gemäß einem der vorhergehenden Ansprüche, dadurch gekennzeichnet, daß das Metallgehäuse (10) eine zylindrische Form besitzt, und daß die in dem Gehäuse angeordnete Abschirmung (13) für elektromagnetische Strahlung aus einer Metallplatte besteht.

9. Vorrichtung gemäß Anspruch 8, dadurch gekennzeichnet, daß sich die Metallplatte (13) in einer diametrischen Ebene des Zylinders befindet und das Innenvolumen des Gehäuses (10) in zwei Halbzylinder unterteilt, wobei der Generator (2) in einem dieser Halbzylinder angeordnet ist, während der Verstärker (6) und die Detektionsmittel (8) in dem anderen Halbzylinder angeordnet sind.
